# FASCICULE DE BREVET EUROPEEN

(11) **EP 1 091 970 B1**
(45) Date de publication et mention de la délivrance du brevet: **21.05.2003**
(21) Numéro de dépôt: 99914703.6
(22) Date de dépôt: 28.04.1999
(51) Int. Cl.: C07H 7/04

(54) **PROCEDE DE PREPARATION D'ALOINE PAR EXTRACTION**
VERFAHREN ZUR HERSTELLUNG VON ALOIN DURCH EXTRAKTION
METHOD FOR PREPARING ALOIN BY EXTRACTION

(43) Date de publication de la demande: 18.04.2001
(73) Titulaire: Paxa N.V., 2585 GB Den Haag (NL)
(72) Inventeur: COHEN, Avraham, 64587 Tel Aviv (IL)
(74) Mandataire: Reuteler, Raymond Werner
(86) Numéro de dépôt international: IB9900771
(87) Numéro de publication internationale: WO00066601

(56) Documents cités:
- EP-A- 0 374 890
- WO-A-87/00052
- US-A- 4 602 004

## Description

La présente invention concerne un procédé de préparation d'aloine, et plus particulièrement un procédé d'extraction d'aloine à partir de sève d'aloe, ou de produits dérivés, utilisable industriellement et procurant un produit final de bonne pureté avec un rendement satisfaisant.

L'aloine est une substance naturelle que l'on peut obtenir suivant les techniques classiques par extraction à partir de gel d'aloès. C'est une substance d'un grand intérêt en raison des propriétés pharmacologiques de plusieurs dérivés utilisables dans divers médicaments. Par exemple, la rhéine, que l'on peut obtenir à partir de l'aloine, présente des propriétés laxatives, antirhumatismales, antiarthrosiques et antiarthritiques utiles en thérapeutique.

Les techniques utilisées le plus couramment depuis de nombreuses années consistent généralement à effectuer une extraction à partir de Hard Gum, c'est-à-dire un résidu durci, résultant de la concentration de la sève par simple chauffage à l'air libre sous pression atmosphérique. L'extraction permet d'obtenir de l'aloine qui peut être purifiée par recristallisation dans un solvant approprié.

Le brevet EP-A-374.890 décrit un procédé d'extraction d'aloine et d'aloe-émodine à partir de plantes ou d'extraits de plantes au moyen d'un solvant hydrophile, filtration et évaporation pour obtenir un sirop sur lequel on effectue ensuite plusieurs extractions, puis une recristallisation. Les solvants utilisés sont des alcools, l'acétone, l'acétate d'éthyle et l'eau.

La dénomination chimique courante de l'aloine est 10-glucopyranosyl-1,8-dihydroxy-3-hydroxyméthyl-9(10H)-anthracénone. L'aloine est présente sous forme de deux isomères A et B, se différenciant par la position du reste glucose sur l'anthrone de base, dont les proportions peuvent varier suivant l'origine des plantes et les procédés d'extraction utilisés. On préfère généralement une aloine à forte teneur en isomère A.

Les études et essais entrepris par la demanderesse ont montré qu'il était possible d'améliorer de manière significative le procédé d'extraction classique en utilisant un additif facilitant l'extraction et permettant ensuite d'amé-liorer la purification. En particulier, le procédé suivant l'invention permet d'améliorer la sélectivité de l'extraction et de réduire considérablement les quantités de solvant utilisées lors de la purification, dans des conditions d'utilisation mises en oeuvre sur le plan industriel.

Le procédé de préparation d'aloine suivant la présente invention comprend essentiellement une extraction d'un exsudat d'aloe, en particulier la sève jaune d'aloe, par exemple, d'aloe vera, d'aloe barbadensis ou encore d'aloe capensis, ou un produit dérivé, en présence d'un diol ou triol aliphatique à bas poids moléculaire, suivie par une purification par cristallisation.

Suivant une forme préférentielle de réalisation, dans une première étape le diol ou triol aliphatique à bas poids moléculaire est ajouté à la sève jaune d'aloe, et on effectue une concentration, puis dans une deuxième étape on réalise une extraction. Bien entendu, cette première étape peut être réalisée sur un produit dérivé de la sève contenant de l'aloine, tel que le Hard Gum, mais il est préférable d'utiliser directement la sève jaune qui correspond à une substance moins dégradée.

L'extraction est suivie d'une recristallisation dans un alcool qui peut être choisi parmi l'éthanol, le n-propanol, l'isopropanol, le n-butanol, le t-butanol et l'isobutanol.

Suivant la présente invention, l'expression "diol ou triol aliphatique à bas poids moléculaire" désigne un diol ou triol ne contenant pas plus de 15 atomes de carbone dans sa chaîne carbonée, et de préférence pas plus de 10 atomes de carbone. Le diol ou triol aliphatique à bas poids moléculaire utilisé dans l'invention peut être par exemple un glycol ou le glycérol. Le glycol est de préférence choisi parmi l'éthylène glycol, le diéthylène glycol, le propylène glycol, le butylène glycol, le tripropylène glycol et le triéthylène glycol. Le diol ou triol aliphatique est utilisé en une quantité comprise entre 5 et 25% en poids, de préférence entre 7 et 15%, par rapport au poids de sève jaune utilisée comme produit de départ.

Comme indiqué ci-dessus, après concentration de la sève jaune en présence de diol ou triol aliphatique, l'extraction est effectuée dans un solvant choisi de préférence parmi l'acétate d'éthyle et l'acétone.

Le choix du solvant d'extraction est important pour obtenir un rendement satisfaisant. Ainsi, des essais comparatifs ont montré que le rendement global par rapport à l'aloine présente dans le produit de départ, est de 50% environ quand on utilise l'acétate d'éthyle, tandis que l'extraction est faible, c'est-àdire inférieure à 10%, si on utilise du chloroforme ou de l'éther de pétrole. De même, la pureté du produit après extraction, c'est-à-dire la teneur en aloine, est inférieure à 10% si l'acétate d'éthyle ou l'acétone est remplacé par du chloroforme.

Le procédé de l'invention apporte une importante amélioration de la sélectivité de l'extraction par comparaison avec les procédés classiques, puisque la teneur en aloine dans le produit extrait avant purification est de l'ordre de 60%, tandis qu'elle est de 50% environ dans le cas d'une extraction par une méthode usuelle.

La maîtrise de la teneur en eau lors de la purification par recristallisation dans un alcool permet d'optimiser la qualité de l'aloine obtenue et le rendement global. Ainsi, suivant une caractéristique avantageuse de l'invention, la purification par recristallisation est effectuée en présence de 0,5 à 5%, de préférence 0,5 à 2%, en poids d'eau par rapport au poids total du milieu. La quantité d'eau peut être ajustée de manière usuelle, soit par addition d'eau si la teneur est trop faible, soit par distillation azéotropique si elle est trop élevée.

Le procédé suivant la présente invention permet d'obtenir un produit dont la teneur en aloine est supérieure à 80%, généralement comprise entre 85 et 90%, et même supérieure à 90% en contrôlant la teneur en eau lors de la purification. Le rendement global par rapport à l'aloine présente dans la substance de départ (sève jaune d'aloe ou produit dérivé) est supérieur à 50%, dans des conditions de mise en oeuvre applicables industriellement.

Un autre avantage du procédé de l'invention est de permettre une diminution importante de la quantité d'alcool utilisée dans l'étape de purification suivant l'extraction. Ainsi, dans le cas d'une recristallisation dans l'isobutanol, la quantité d'alcool est comprise entre 10 et 20 volumes pour un volume d'aloine présente dans le milieu, contre 40 à 60 volumes dans les procédés usuels.

L'aloine obtenue par le procédé de l'invention peut être utilisée pour la préparation de médicaments à base de rhéine ou de dérivés, utilisés dans le traitement de l'arthrose, l'arthrite et diverses inflammations.

Les exemples suivants illustrent l'invention plus en détail sans en limiter la portée. Sauf indication contraire, les parties et pourcentages sont indiqués en poids.

### Exemple 1

A titre d'exemple comparatif, on prépare de l'aloine par la technique indiquée ci-après.

Dans un ballon évaporateur de 1 1, on concentre 200 g de sève jaune d'aloe vera sur bain d'eau chauffé à environ 55°C, sous une pression d'environ 4.10³ Pa. Après élimination de 75% d'eau, le concentré est repris sous forte agitation pendant 30 minutes par 600 ml d'acétate d'éthyle à une température comprise entre 55 et 60°C.

Après décantation, la phase organique surnageante est soutirée et le résidu aqueux est extrait 3 fois à l'acétate d'éthyle à une température comprise entre 50 et 60°C. Les solutions d'extraction sont réunies et concentrées sous vide en maintenant la température entre 45 et 50°C.

On obtient ainsi un produit solide de couleur brun-orangé, dont la teneur en aloine est d'environ 45-50%, que l'on redissout dans 640 ml d'isobutanol à 70°C (soit 40 volumes d'isobutanol par rapport à l'aloine présente).

La solution isobutanolique est refroidie à 5°C et maintenue à cette température pendant environ 4 heures.

L'aloine qui cristallise est recueillie par filtration sur Büchner et le gâteau est lavé par 20 ml d'isobutanol.

Après séchage, on obtient ainsi un produit sous forme de poudre de couleur jaune-brun dont la teneur en aloine, déterminée par chromatographie HPLC, est de 86%.

Le rendement en aloine pure par rapport à l'aloine de la sève jaune de départ est de 40%.

### Exemple 2

Le procédé suivant la présente invention reprend le principe de l'extraction décrit dans l'Exemple 1, modifié comme indiqué ci-après.

Dans un ballon évaporateur de 1 1, on introduit 200 g de sève jaune d'aloe vera identique à celle utilisée dans l'Exemple 1, à laquelle on a préalablement ajouté 20 g d'éthylène glycol, et on concentre sous une pression d'environ 4.10³ Pa. Après élimination de 75% d'eau, le concentré est repris par 600 ml d'acétate d'éthyle dans les mêmes conditions que dans l'Exemple 1.

Après décantation, la phase organique surnageante est soutirée et le résidu aqueux est extrait 3 fois à l'acétate d'éthyle, puis les solutions d'extraction sont réunies et concentrées sous vide en maintenant la température entre 45 et 50°C.

On obtient ainsi un produit huileux de couleur brune, dont la teneur en aloine est d'environ 55-60%, que l'on reprend dans 240 ml d'isobutanol à 70°C (soit 15 volumes d'isobutanol par rapport à l'aloine présente).

La solution isobutanolique est refroidie à 5°C et maintenue à cette température pendant environ 4 heures, comme dans l'Exemple 1.

L'aloine qui cristallise est recueillie par filtration sur Büchner et, après lavage par 20 ml d'isobutanol, et séchage, on obtient ainsi un produit sous forme de poudre de couleur jaune-brun dont la teneur en aloine, déterminée par chromatographie HPLC, est de 87%. Le rapport de l'aloine A à l'aloine B est d'environ 3/1.

Le rendement en aloine pure par rapport à l'aloine de la sève jaune de départ est de 53%.

### Exemple 3

On procède comme indiqué dans l'Exemple 2, mais en remplaçant l'éthylène glycol par le diéthylène glycol en même quantité.

On obtient ainsi une aloine pure à 87% avec un rendement de 52%.

### Exemple 4

On procède comme dans l'Exemple 2, mais en remplaçant l'éthylène glycol par le glycérol en même quantité.

On obtient ainsi un produit dont la teneur en aloine est égale à 87%, avec un rendement global de 48%.

### Exemple 5

On procède comme dans l'Exemple 2, mais après extraction à l'acétate d'éthyle, l'extrait obtenu est repris par 240 ml d'isobutanol tout en ajustant la quantité d'eau à 1% par rapport au poids total du milieu.

On obtient ainsi un produit dont la teneur en aloine est égale à 90%, avec un rendement global de 56%.

## Revendications

1. Procédé de préparation d'aloine à partir d'une substance contenant de l'aloe, par extraction et purification par cristallisation, **caractérisé en ce que** l'on effectue une extraction sur la sève jaune d'aloe ou son sésidu durci, en présence d'un diol ou triol aliphatique ne contenant pas plus de 15 atomes de carbone dans la chaîne carbonée

2. Procédé selon la revendication 1, **caractérisé en ce que** le diol ou triol aliphatique ne contenant pas plus de 15 atomes de carbone dans la chaîne carbonée est ajouté à la sève jaune d'aloe, puis on effectue une concentration avant extraction.

3. Procédé selon l'une quelconque des revendication précédentes, **caractérisé en ce que** l'extraction est suivie d'une recristallisation dans un alcool.

4. Procédé selon la revendication 3, **caractérisé en ce que** l'alcool est choisi parmi l'éthanol, le n-propanol, l'isopropanol, le n-butanol, le t-butanol et l'isobutanol.

5. Procédé selon la revendication 1, **caractérisé en ce que** l'extraction est effectuée sur la sève d'aloe vera, d'aloe barbadensis ou d'aloe capensis.

6. Procédé selon la revendication 1, **caractérisé en ce que** le diol ou triol aliphatique ne contenant pas plus de 15 atomes de carbone dans la chaîne carbonée est un glycol ou le glycérol.

7. Procédé selon la revendication 6, **caractérisé en ce que** le glycol est choisi parmi l'éthylène glycol, le diéthylène glycol, le propylène glycol, le butylène glycol, le tripropylène glycol et le triéthylène glycol.

8. Procédé selon l'une quelconque des revendications 4 à 7, **caractérisé en ce que** la quantité de diol ou triol aliphatique ne contenant pas plus de 15 atornes de carbone dans sa chaîne carbonée est comprise entre 5 et aliphatique est comprise entre 5 et 25% en poids par rapport au poids de sève jaune.

9. Procédé selon l'une quelconque des revendications 1 à 3, **caractérisé en ce que** l'extraction est effectuée dans un solvant choisi parmi l'acétate d'éthyle et l'acétone.

10. Procédé selon l'une quelconque des revendications 1 à 3, **caractérisé en ce que** la recristallisation est effectuée en présence de 0,5 à 5% en poids d'eau par rapport au poids total du milieu.

## Patentansprüche

1. Verfahren der Aloinherstellung, von einer Aloe enthaltenden Substanz ausgehend, durch Extraktion und Reinigung durch Umkristallisieren, **dadurch gekennzeichnet, dass** eine Extraktion am gelben Saft von Aloe oder seinem gehärteten Rückstand in Gegenwart eines aliphatischen Diols oder Triols, das nicht mehr als 15 Kohlenstoffatome in seiner Kohlenstoffkette hat, ausgeführt wird.

2. Verfahren gemäss Anspruch 1, **dadurch gekennzeichnet, dass** das aliphatische Diol oder Triol, das nicht mehr als 15 Kohlenstoffatome in seiner Kohlenstoffkette hat, dem gelben Aloesaft zugegeben wird und dann eine Konzentration vor der Extraktion ausgeführt wird.

3. Verfahren gemäss einem beliebigen der vorangehenden Ansprüche, **dadurch gekennzeichnet, dass** der Extraktion eine Umkristallisation aus einem Alkohol folgt.

4. Verfahren gemäss Anspruch 3, **dadurch gekennzeichnet, dass** der Alkohol unter Ethanol, *n*-Propanol, Isopropanol, *n*-Butanol, *t*-Butanol und Isobutanol ausgewählt wird.

5. Verfahren gemäss Anspruch 1, **dadurch gekennzeichnet, dass** die Extraktion am Saft von *aloe vera*, von *aloe barbadensis* oder von *aloe capensis* ausgeführt wird.

6. Verfahren gemäss Anspruch 1, **dadurch gekennzeichnet, dass** das aliphatische Diol oder Triol, das nicht mehr als 15 Kohlenstoffatome in seiner Kohlenstoffkette hat, ein Glykol oder das Glycerin ist.

7. Verfahren gemäss Anspruch 6, **dadurch gekennzeichnet, dass** das Glykol unter Ethylenglykol, Diethylenglykol, Propylenglykol, Butylenglykol, Tripropylenglykol und Triethylenglykol ausgewählt wird.

8. Verfahren nach einem beliebigen der Ansprüche 4 bis 7, **dadurch gekennzeichnet, dass** die Menge von aliphatischem Diol oder Triol, das nicht mehr als 15 Kohlenstoffatome in seiner Kohlenstoffkette hat, zwischen 5 und 25 Gewichtsprozent bezogen auf das Gewicht des gelben Saftes beträgt.

9. Verfahren gemäss einem beliebigen der Ansprüche 1 bis 3, **dadurch gekennzeichnet, dass** die Extraktion in einem zwischen Ethylacetat und Aceton ausgewählten Lösungsmittel durchgeführt wird.

10. Verfahren gemäss einem beliebigen der Ansprüche 1 bis 3, **dadurch gekennzeichnet, dass** die Umkristallisation in Gegenwart von 0,5 bis 5 Gewichtsprozent von Wasser bezogen auf das Gesamtgewicht des Mediums durchgerührt wird.

## Claims

1. Method for preparing aloin from a substance containing aloe, by extraction and purification by crystallization, **characterized in that** one performs an extraction of the yellow sap of aloe or of its hard gum in the presence of an aliphatic diol or triol with no more than 15 carbon atoms in its carbon chain.

2. Method according to claim 1, **characterized in that** the aliphatic diol or triol is added to the yellow sap of aloe, then a concentration is performed prior to extraction.

3. Method according to any of the preceding claims, **characterized in that** the extraction is followed by a recrystallization in an alcohol.

4. Method according to claim 3, **characterized in that** the alcohol is selected from among ethanol, n-propanol, isopropanol, n-butanol, tert-butanol, and isobutanol.

5. Method according to claim 1, **characterized in that** the extraction is performed on the sap of aloe vera, aloe barbadensis or aloe capensis.

6. Method according to claim 1, **characterized in that** the aliphatic diol or triol is a glycol or glycerol.

7. Method according to claim 6, **characterized in that** the glycol is selected from among ethylene glycol, diethylene glycol, propylene glycol, butylene glycol, tripropylene glycol, and triethylene glycol.

8. Method according to any of claims 4 to 7, **characterized in that** the amount of aliphatic diol or triol is between 5 and 25% by weight relative to the weight of yellow sap.

9. Method according to any of claims 1 to 3, **characterized in that** the extraction is performed with a solvent selected among ethyl acetate and acetone.

10. Method according to any of claims 1 to 3, **characterized in that** the recrystallization is performed in the presence of 0.5 to 5% by weight of water relative to the medium's total weight.
